# EUROPEAN PATENT APPLICATION

(11) **EP 4 846 957 A1**
(43) Date of publication of application: **02.12.2026**
(21) Application number: 26181236.6
(22) Date of filing: 27.05.2026
(51) Int. Cl.: A61B 17/32, A61B 17/3209, A61F 11/20, A61N 1/05, A61N 1/36, A61N 1/372, A61B 17/00

(54) **SURGICAL TOOL FOR IMPLANTING A COCHLEAR IMPLANT ELECTRODE INTO THE SCALA TYMPANI OF THE INNER EAR**

(30) Priority: 27.05.2025 PL 45216025
(71) Applicant: Instytut Fizjologii I Patologii Sluchu, 02-042 Warszawa (PL)
(72) Inventor: Skarzynski, Henryk, 01-494 Warszawa (PL); Skarzynski, Piotr Henryk, 01-494 Warszawa (PL)
(74) Representative: Godlewski, Piotr

(57) **Abstract**

A surgical tool for implantation of a cochlear implant electrode into the scala tympani of the inner ear, comprising a tip (1), a shank (2), and an ergonomic handle (3), is characterized in that the tip (1) is formed as a solid body having a shape approximating a cone with a height (WO) in the range of 1.0-2.0 mm, and having a base in the form of a regular figure whose surface is bounded by two arcs (AB, BA) of equal length connected at points, the tip having a major diameter (AB) with a length in the range of 0.8-1.4 mm and a minor diameter (CD) with a length in the range of 0.2-0.3 mm. Blades are preferably profiled along the left generatrix WA and the right generatrix WB. The shank (2) is preferably formed as an elongated truncated cone having a lower base diameter in the range of 2.0-5.0 mm, uniformly tapering from the handle (3) toward the tip (1) and having an upper base diameter in the range of 0.4-0.8 mm.

## Description

The invention relates to a surgical tool used in procedures to implant cochlear implants with electrode lengths ranging from 10 mm to 34 mm, whilst preserving the integrity of the inner ear and maintaining pre-operative hearing at low or high frequencies.

There are known treatments for deafness, involving the surgical implantation of cochlear implants.

To the best of our knowledge, there is a cochlear surgery technique known as 'Skarz̋yński's 4-step method in cochlear implant surgery'. In this method, the first step involves making a 16-20 mm incision in the skin behind the ear in the shape of an elongated S, performing a limited opening of the mastoid cavity and creating an approach allowing access and visualization of the tympanic cavity in the region of the round window niche. The second step involves exposing the membrane of the round window by removing the bony overhangs that naturally cover it, in a manner tailored to each patient. The next step is to make a small puncture in the window membrane, followed by a longitudinal incision from front to back, along a horizontal line. In the third step, an active electrode of the cochlear implant, of a suitably selected length, is inserted at as close to a right angle as possible to the surface of the round window membrane, whilst simultaneously measuring the response from the inner ear, which reflects the functional status of the preserved, intact part of the ear responsible for receiving acoustic stimuli. The third step, however, involves securing the active electrode in place, sealing the physiological opening in the round window, positioning the remaining electrode within the mastoid cavity, and placing the inner coil in a pocket created on the surface of the squamous part of the temporal bone beneath the musculocutaneous-periosteal flap. The fourth step involves closing the incision made in the skin and subcutaneous tissues at the outset, using a two-layer technique comprising subcutaneous and skin sutures, and joining the skin layers using tissue adhesive or a combination of both methods.

This method ensures minimally invasive exposure of the round window membrane and the implantation of the active electrode of the cochlear implant into the scala tympani of the inner ear within the appropriate range. At the same time, this allows the structure of the inner ear to be preserved, which may make it possible, in an unpredictable future, to use other, more modern and effective solutions or to replace the implant non-invasively.

The following tools for surgical ear procedures are known in the art; however, they are of little practical use for making the appropriate incision in the round window membrane with minimal disruption to the fluid levels in the scala tympani, in accordance with Skarźyński's method.

The suction knife for myringotomy, described in utility model CN203898546U, consists of a working rod and a suction tube. The working rod is tubular; one end of the working rod is connected to one end of the suction tube. The end face of the other end of the suction tube is a bevelled end face, and the curved cutting edge is formed at the lower part of the bevelled end face. The cleaning needle is mounted so that it can move within the working rod and the suction tube. This tool can be used to make a direct incision in the eardrum, creating a linear incision. The incision is short, and the fluid from the middle ear can be aspirated directly whilst the eardrum is being incised; this constitutes an invasive disruption of the inner ear's fluid balance and poses a very significant risk to the preservation of pre-operative hearing.

An ear surgery tool is described in utility model CN212308205U. The tool comprises a handle portion, an insertion portion, and a venting device movably arranged on the insertion portion. In the insertion portion, at least one section of a suction tube and at least one section of a pushing tube are contained within an outer sleeve. The pushing tube and the tube body are arranged sequentially on different sections of the outer side wall of the suction tube. The suction tube is connected to the interior of the tube body to form a suction channel.

A disposable knife for preparing the skin prior to endoscopic ear surgery is described in utility model CN209645221U. The scalpel consists of a blade, a shaft and a handle. The scalpel head is fan-shaped and is located at one end of the body, whilst a suction opening is formed in the body near the head, the handle is located at the other end of the body, whilst a cavity is formed in the body and handle, which is connected to the suction port and linked to the vacuum aspirator connector.

The main components of the tool according to the invention are the tip, the shank and the ergonomic handle. The invention is characterised by the fact that the tip is formed as a solid body resembling a cone with a height in the range of 1.0-2.0 and a base in the form of a regular figure, the surface of which is bounded by two arcs of equal length joined at a single point. The base has a large diameter ranging from 0.8 to 1.4 mm and a small diameter ranging from 0.2 to 0.3 mm. Blades are advantageously profiled along the left-hand and right-hand outer edges of the body. The angle between the symmetry axis of the shank and the symmetry axis of the handle is in the range of 150°-170°. The angle between the symmetry axis of the tip and the symmetry axis of the shank is 110°-120° in the left-hand version and 240°-250° in the right-hand version. At the same time, the angle between the symmetry axis of the shank and a straight line on which the major diameter of the base lies is in the range of 74°-76°. The shank is preferably formed as an elongated truncated cone having a lower base diameter in the range of 2.0-5.0 mm, tapering uniformly toward the tip and having an upper base diameter in the range of 0.4-0.8 mm.

In this embodiment of the invention, the end of the handle is of the pencil type.

In another embodiment, a finger grip is moulded onto the end of the handle.

The tool described in the invention ensures that surgical procedures within the middle ear are carried out efficiently and accurately. In particular, the tool enables safe opening of the scala tympani by allowing an incision to be made at an appropriate angle and by guiding it in a plane properly oriented with respect to the surface of the round window membrane. The tool ensures that the active electrode of a minimally invasive cochlear implant is inserted into the cochlear canal to the correct depth, whilst maintaining the necessary flexibility. This is a particularly advantageous feature of the device, as it ensures that the structure of the ear is preserved as much as possible during the treatment of partial deafness, whilst maintaining pre-operative hearing, whether good, functionally adequate, or impaired but capable of being amplified, in the low or high frequency ranges.

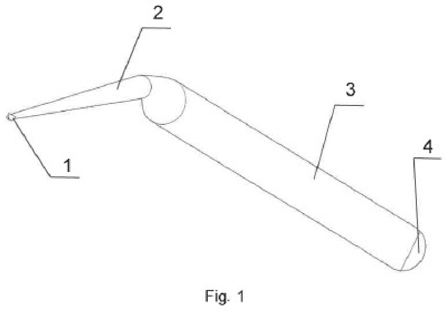

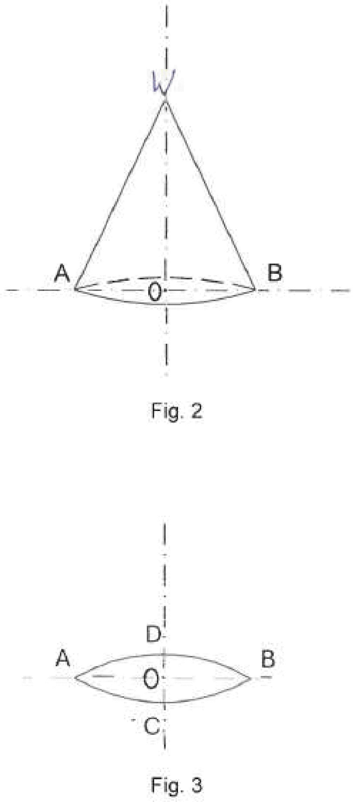

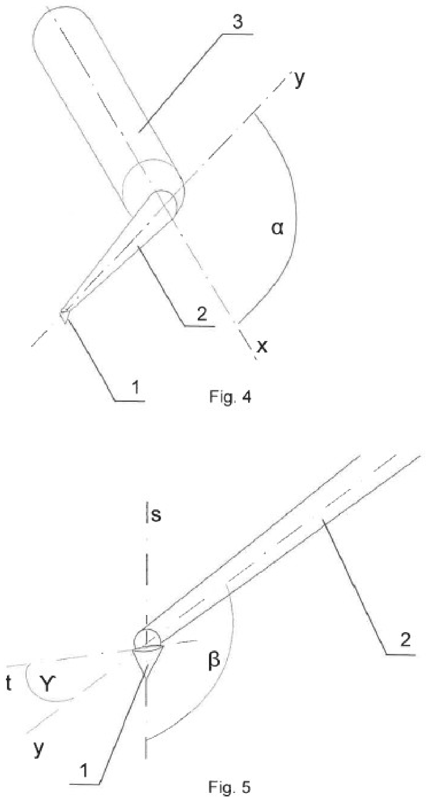

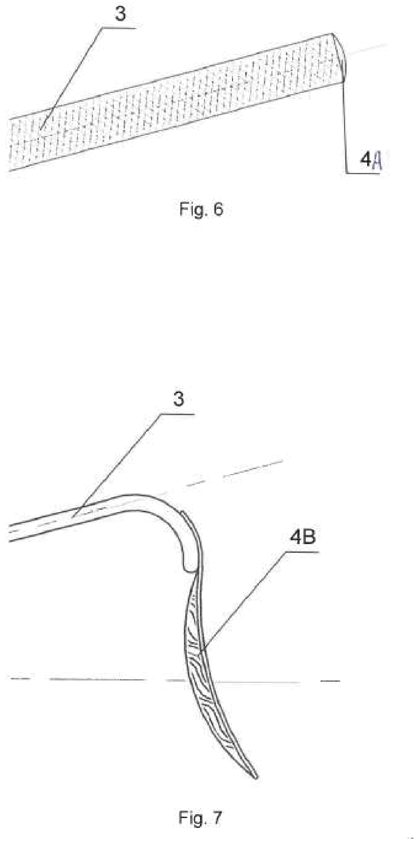

The invention is further explained in the embodiment and in the drawing, in which Fig. 1 schematically shows the tool in a general view, Fig. 2 shows a projection of the tip onto a plane, Fig. 3 shows the shape of the base of the tip, Fig. 4 shows the spatial relationship between the tip, the shank and the handle, Fig. 5 shows a detailed spatial relationship between the tip and the shank, and Figs. 6 and 7 show two variants of the handle end.

### Example 1

A surgical tool for implantation of a cochlear implant electrode into the scala tympani of the inner ear comprises a tip (1), a shank (2), and an ergonomic handle (3), the distal end (4A) of which is pencil-shaped. The tip (1) is formed as a solid body having a shape approximating a cone with a height (WO) of 1.4 mm and having a base in the form of a regular figure, the surface of which is bounded by an arc (AB) connected at points to an arc (BA). The arc AB and the arc BA have equal lengths.

The base figure has a major diameter (AB) with a length of 1.1 mm and a minor diameter (CD) with a length of 0.3 mm. Blades are moulded into the body along the left generatrix WA and along the right generatrix WB. The angle (α) between the symmetry axis (y) of the shank (2) and the symmetry axis (x) of the handle (3) is 165°. The angle (γ) between the symmetry axis (y) of the shank (2) and the straight line (t) on which the major diameter (AB) lies is 75°.

The shank (2) has a length of 5.5 mm and is formed as an elongated truncated cone expanding in the direction of the handle (3), from a lower base diameter of 1.0 mm to an upper base diameter of 3.0 mm.

The ergonomic handle (3), having a diameter of 6 mm and a length of 10 cm, is provided with a pencil-shaped end portion (4A) and a knurled side surface.

The tool is made of hardened stainless steel or titanium.

### Example 2

The tool is as in Example 1, except that the angle (β) between the symmetry axis (s) of the tip (1) and the symmetry axis (y) of the shank (2) is 245°, and the cylindrical portion of the handle has a diameter of 3 mm and a length of 55 mm. The end portion (4B) of the handle (3) is formed as a fingertip grip.

## Claims

1. A surgical tool for inner ear surgery, comprising a tip, a shank, and an ergonomic handle, **characterized in that** the tip (1) is formed as a solid body having a shape approximating a cone with a height (WO) in the range of 1.0-2.0 mm, and having a base in the form of a regular figure whose surface is bounded by two arcs (AB, BA) of equal length connected at points, the tip having a major diameter (AB) with a length in the range of 0.8-1.4 mm and a minor diameter (CD) with a length in the range of 0.2-0.3 mm, and wherein, along the left generatrix (WA) and along the right generatrix (WB), blades are preferably formed, and the angle (α) between the symmetry axis (y) of the shank (2) and the symmetry axis (x) of the handle (3) is in the range of 150°-170°, and the angle (β) between the symmetry axis (s) of the tip (1) and the symmetry axis (y) of the shank (2) in a left-hand version is in the range of 110°-120°, while in a right-hand version it is in the range of 240°-250°, and simultaneously the angle (y) between the symmetry axis (y) of the shank (2) and a straight line (t) on which the major diameter (AB) lies is in the range of 74°-76°, wherein the shank (2) is preferably formed as an elongated truncated cone having a lower base diameter in the range of 2.0-5.0 mm, tapering uniformly from the handle (3) toward the tip (1) and having an upper base diameter in the range of 0.4-0.8 mm.

2. The tool according to claim 1, **characterised in that** the end (4A) of the handle (3) is of the pencil type.

3. The tool according to claim 1, **characterised in that** a finger grip is formed at the end (4B) of the handle (3).
